# EUROPEAN PATENT SPECIFICATION

(11) **EP 4 498 912 B1**
(45) Date of publication and mention of the grant of the patent: **06.08.2025**
(21) Application number: 23719990.6
(22) Date of filing: 31.03.2023
(51) Int. Cl.: A61M 39/24, A61M 39/10, A61B 5/15, A61B 5/153

(54) **HEMOLYSIS-REDUCTION ACCESSORIES FOR DIRECT BLOOD DRAW**
HÄMOLYSEREDUKTIONSZUBEHÖR ZUR DIREKTEN BLUTENTNAHME
ACCESSOIRES DE RÉDUCTION D'HÉMOLYSE POUR PRISE DE SANG DIRECTE

(30) Priority: 12.05.2022 US 202263341418 P
(43) Date of publication of application: 05.02.2025
(60) Divisional application: 25186150.6
(73) Proprietor: CareFusion 303, Inc., San Diego, CA 92130 (US)
(72) Inventor: JADHAV, Amarsinh Deeliprao, Karnataka Bengaluru 560045 (IN); KHAN, Mohammed Mehtab, Karnataka Bengaluru 560045 (IN); K, Kowshika, Karnataka Bengaluru 560045 (IN)
(74) Representative: Zenz Patentanwälte Partnerschaft mbB
(86) International application number: PCT/US2023/017125
(87) International publication number: WO 2023/219726

(56) References cited:
- WO-A1-2014/204586
- US-A- 4 244 378
- US-A1- 2013 178 760
- US-A1- 2019 076 074
- US-A1- 2021 128 037

## Description

### CROSS-REFERENCE TO RELATED APPLICATIONS

This application claims the benefit of U.S. Provisional Application No. 63/341,418, titled "HEMOLYSIS-REDUCTION ACCESSORIES FOR DIRECT BLOOD DRAW," filed May 12, 2022.

### TECHNICAL FIELD

The present disclosure generally relates to blood draw and administration of parenteral fluids to a patient, and particularly to systems and non claimed methods to reduce hemolysis in PIVC blood draw using a flow restriction device.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is an over-the-needle peripheral intravenous ("IV") catheter (PIVC). As its name implies, the over-the-needle catheter may be mounted over an introducer needle having a sharp distal tip. A catheter assembly may include a catheter hub, the catheter extending distally from the catheter hub, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe. Alternatively, the blood collection container may include a test tube with a rubber stopper at one end. In some instances, the test tube has had all or a portion of air removed from the test tube so pressure within the test tube is lower than ambient pressure. Such a blood collection container is often referred to as an internal vacuum or a vacuum tube. A commonly used blood collection container is a VACUTAINER^{®} blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops. In some instances, blood is drawn into the collection container by withdrawing the plunger of a syringe.

Unfortunately, as blood is drawn into the blood collection container, red blood cells are in a high shear stress state and susceptible to hemolysis due to a high initial pressure differential between the vein and the blood collection container. Hemolysis may result in rejection and discard of a blood sample. The high initial pressure differential can also result in catheter tip collapse, vein collapse, or other complications that prevent or restrict blood from filling the blood collection container. Furthermore, blood spillage during and/or after blood draw commonly occurs.

In some instances, withdrawing a plunger of a syringe coupled to the catheter may exert an unintended pressure on the blood such that the red blood cells are in a high shear stress state, which may result in hemolysis. Further, the process of withdrawing the plunger of a syringe to draw blood into the blood collection container is reliant upon the control and skill applied by the user, which may vary among different users.

The description provided in the background section should not be assumed to be prior art merely because it is mentioned in or associated with the background section. The background section may include information that describes one or more aspects of the subject technology.

WO 2014/204586 A1 discloses a bidirectional valve for medical use or the like providing different threshold opening pressures in different directions using a valve seat and flapper construction providing improved characterization in operation in contrast to cross-slit valves often used for bidirectional operation. A valve disk supported in a central annular region provides movable portions engaging in valve seats at a peripheral region and a central region.

### SUMMARY

The invention is defined in the independent claims. Preferable embodiments are further laid out in the dependent claims. The present disclosure provides a flow restriction device which is not according to the present invention, comprising: a proximal housing comprising a first end portion forming a proximal port, a second end portion, and a passage extending through the first and second end portions; a distal housing comprising a first end portion, a second end portion forming a distal port, and a passage extending through the first and second end portions; a flow insert positioned in a cavity formed between the proximal housing and the distal housing, the flow insert comprising a fluid passage extending therethrough, the fluid passage comprising a first segment formed by an outer surface of the flow insert, and a second segment formed by an inner surface of the flow insert; a resilient valve positioned between the outer surface of the flow insert and the distal housing, the resilient valve comprising a first end having an inner portion and an outer portion, the inner portion aligned with the second segment of the fluid passage of the flow insert, and the outer portion aligned with the first segment of the fluid passage of the flow insert; wherein the inner portion of the resilient valve is flexible, relative to the proximal housing, such that in a first position of the resilient valve, the inner portion is spaced apart from the flow insert by a first distance to permit a fluid to move between the first and second segments of the fluid passage, and in a second position, the inner portion is spaced apart from the flow insert by a second distance to resist movement of the fluid between the first and second segments of the fluid passage.

The present invention provides a flow restriction device according to claim 1, comprising: a flow insert comprising a fluid passage extending therethrough, the fluid passage comprising a first segment formed by an outer surface of the flow insert, and a second segment formed by an inner surface of the flow insert; a resilient valve comprising a first end engaged against the outer surface of the flow insert, the first end having an inner portion aligned with the second segment of the fluid passage, and an outer portion aligned with the first segment of the fluid passage, and an aperture that extends through the resilient valve, from the first end to a second end of the resilient valve; wherein the inner portion of the resilient valve is flexible, relative to the flow insert such that in a first orientation of the resilient valve, the inner portion is spaced apart from the flow insert g by a first distance to permit a fluid to move from the first segment toward the second segment of the fluid passage, and in a second orientation of the resilient valve, the inner portion is spaced apart from the flow insert by a second distance to resist movement of the fluid from the first segment toward the second segment of the fluid passage.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

FIG. 1 illustrates a vascular access device including a peripheral intravenous catheter (PIVC) assembly that includes a flow restriction device, in accordance with some embodiments of the present disclosure.
FIG. 2 illustrates a perspective view of the flow restriction device, in accordance with some embodiments of the present disclosure.
FIG. 3 illustrates an exploded view of the flow restriction device of FIG. 2, in accordance with some embodiments of the present disclosure.
FIG. 4 illustrates a perspective view of an insert body for a flow restriction device, in accordance with some embodiments of the present disclosure.
FIG. 5 illustrates a cross-sectional view of the flow restriction device of FIG. 2 along line 5-5, in accordance with some embodiments of the present disclosure.
FIG. 6 illustrates a perspective view of a resilient valve for a flow restriction device, in accordance with some embodiments of the present disclosure.
FIG. 7 illustrates a cross-sectional view of the resilient valve of FIG. 7 along line 7-7, in accordance with some embodiments of the present disclosure.
FIG. 8 illustrates a perspective view of a flow insert for a flow restriction device, in accordance with some embodiments of the present disclosure.
FIG. 9 illustrates a cross-sectional view of a flow restriction device, in accordance with some embodiments of the present disclosure.
FIG. 10 illustrates a perspective view of a flow insert for a flow restriction device, in accordance with some embodiments of the present disclosure.
FIG. 11 illustrates a cross-sectional view of a flow restriction device, in accordance with some embodiments of the present disclosure.

### Detailed Description

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Blood draw via a vascular access device has drawn increasing attention attributed to minimized needle sticks and improved operation efficiency as compared with traditional blood draw methods with venipuncture. Current blood draw using a peripheral intravenous catheter (PIVC) has seen some challenges, one of the most critical is hemolysis related blood quality. In particular, with currently existing PIVC products in the market, along with the standard connection (such as a short extension set and a needleless connector), and blood collection devices (such as a Vacutainer), the shear stress exerted onto blood cells tends to be on the verge of hemolyzing.

Various embodiments of the present disclosure are directed to providing systems and non claimed methods to address hemolysis in PIVC blood draw with a hemolysis reduction accessory (also referred to herein as a flow restriction device) which can be pre-attached to the PIVC and decrease a flow rate to reduce a risk of hemolysis. The hemolysis-reduction accessory is advantageously compatible with PIVC placement and does not necessitate change to any of the existing operations. The hemolysis-reduction accessory of the various embodiments described herein is potentially applicable to a wide variety of PIVC products, and compatible with existing blood collection devices and infusion disposables.

Various embodiments of the present disclosure focus on effective flow rate reduction with the add-on hemolysis-reduction accessory (also referred to herein as a flow restriction device) that regulates the overall flow rate of the entire fluid path as blood cells travel through. The flow restriction device can be either assembled with the PIVC or co-packaged with the PIVC. As such, there is no additional operation during catheter placement since the device can permit blood flashback. The clinician may connect a blood collection device to a port or opening of the accessory and can then draw blood to the intended volume. After blood draw, the clinician may disconnect and discard the flow restriction device and the blood collection device together. As such, this flow restriction device can be either for single blood draw or stay inline throughout indwell.

The flow restriction devices and associated blood collection systems of the various embodiments described herein additionally provide further advantages over currently existing blood collection systems. For example, flow restriction devices described herein allow for hemolysis-reduction function to be integrated for PIVC blood draw. Further, the flow restriction devices described herein are compatible with PIVC placement and allow for seamless blood draw at insertion. Additionally, since the flow restriction devices are an add-on which can be easily incorporated without any changes to existing PIVC, there is minimal impact to clinical setting and operations. The present disclosure also provides flow restriction devices and associated blood collection systems that can prevent the hemolysis of blood due to high suction pressure by reducing a cross-sectional area or closing a blood flow path to reduce the flow rate of the blood moving therethrough when the suction pressure goes beyond desired value or range. When the suction pressure returns to a desired value or range, the features of the present disclosure can permit the flow rate of the blood through the blood flow path to increase.

Flow restriction devices of the present disclosure can include a surface forming a fluid passage, and a resilient valve positioned adjacent to or along at least a portion of the fluid passage. At least a portion of the resilient valve is movable or can bias toward or away from the fluid passage. In a first orientation or position of the resilient valve, a fluid such as blood can move along a fluid flow path that includes the surface forming the fluid passage, and between the surface and the resilient valve. In a second orientation or position of the resilient valve, at least a portion of the valve is moved or biased toward the surface forming the fluid passage such that a cross-sectional area of the fluid flow path between the surface and the resilient valve is decreased, relative to the first orientation. In the second orientation, movement of the fluid along the fluid flow path between the surface forming the fluid passage and the resilient valve is reduced or stopped, relative to the first orientation.

In some embodiments of the present disclosure, flow restriction devices can include a surface forming a fluid passage having a first segment and a second segment, and a resilient valve positioned adjacent to or between the first and second segments of the fluid passage, such that a fluid flow path extends between the resilient valve and the surface forming the fluid passage. In a first orientation of the resilient valve, a fluid can move along the fluid flow path between the first and second segments of the fluid passage, and in a second orientation, at least a portion of the valve is moved or biased toward the surface forming the fluid passage such that movement of the fluid along the fluid flow path between the first and second segments is reduced, relative to when the resilient valve is in the first orientation. In some embodiments of the present disclosure, movement of the fluid along the fluid flow path between the first and second segments is obstructed when the resilient valve is in the second orientation.

In some aspects of the present disclosure, flow restriction devices can include housing forming a cavity and at least a portion of the fluid flow path. The surface forming the fluid passage can be formed by a surface of the housing, such as an inner surface, and the resilient valve can be positioned in a cavity of the housing. In some embodiments, an inner surface of the housing can define the fluid passage and the cavity, and the resilient valve is positioned in a cavity. Some embodiments of the present disclosure include a housing having an inner surface forming a cavity, a resilient valve positioned within the cavity, and a flow insert positioned within the cavity and having a surface forming at least apportion of the fluid passage.

The fluid passage or a portion thereof can extend in a direction that is linear or non-linear. In some embodiments, any of a first or second segment of the fluid passage extends in direction that is linear, and the other of the first or second segment of the fluid passage extends in a direction that is non-linear. The non-linear direction can, for example, form a spiral or involute shape. The spiral or involute shape fluid passage can form a first segment portion of the fluid passage that extends radially inward to a second segment of the fluid passage. The spiral or involute shape of the fluid passage can extend around a longitudinal axis through a central portion of the second segment of the fluid passage.

In some embodiments of the present disclosure, the fluid passage or a portion thereof can form an angle between segments, where the angle can be between approximately 0 degrees and approximately 180 degrees. In some embodiments, the angle between segments is approximately 90 degrees.

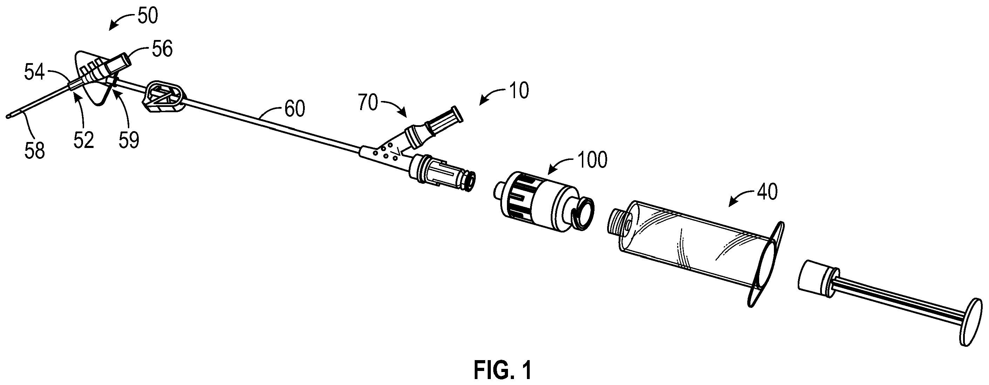

FIG. 1 illustrates a vascular access device 10 including a peripheral intravenous catheter (PIVC) assembly 50, a flow restriction device 100, and a blood collection device 40, in accordance with some embodiments of the present disclosure. The devices illustrated in FIG. 1 can be used, for example, in a syringe blood draw procedure. The flow restriction device 100 may be configured to reduce a likelihood of hemolysis during blood collection using the vascular access device 10 and the blood collection device 40. In some embodiments, the vascular access device 10 may include a catheter assembly 50. The catheter assembly 50 may include a catheter hub 52, which may include a distal end 54, a proximal end 56, and a lumen extending through the distal end and the proximal end. The catheter assembly 50 may further include a catheter 58, which may be secured within the catheter hub 52 and may extend distally from the distal end 54 of the catheter hub 52. In some embodiments, the catheter assembly 50 may be a peripheral intravenous catheter (PIVC).

In some embodiments, the catheter assembly 50 may include or correspond to any suitable catheter assembly 50. In some embodiments, the catheter assembly 50 may be integrated and include an extension tube 60, which may extend from and be integrated with a side port 59 of the catheter hub 52. A non-limiting example of an integrated catheter assembly is the BD NEXIVA^{™} Closed IV Catheter system, available from Becton Dickinson and Company. In some embodiments, a proximal end of the extension tube 60 may be coupled to an adapter, such as, for example, a Y-adapter 70. In some embodiments, the flow restriction device 100 may be fluidly coupled to the Y-adapter 70.

In some embodiments, the catheter assembly 50 may be non-integrated and may not include the extension tube 60. In these and other embodiments, the flow restriction device 100 may be configured to couple to the proximal end 56 of the catheter hub 52 or another suitable portion of the catheter assembly 50. In some embodiments, the flow restriction device 100 may be coupled directly to the catheter assembly 50, eliminating the extension tube 60 and providing a compact catheter system.

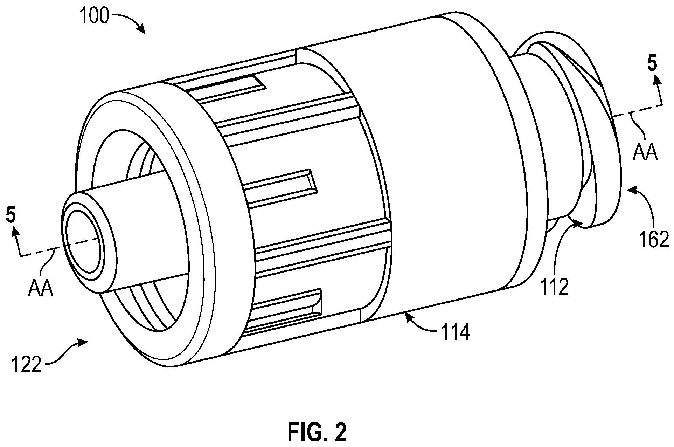

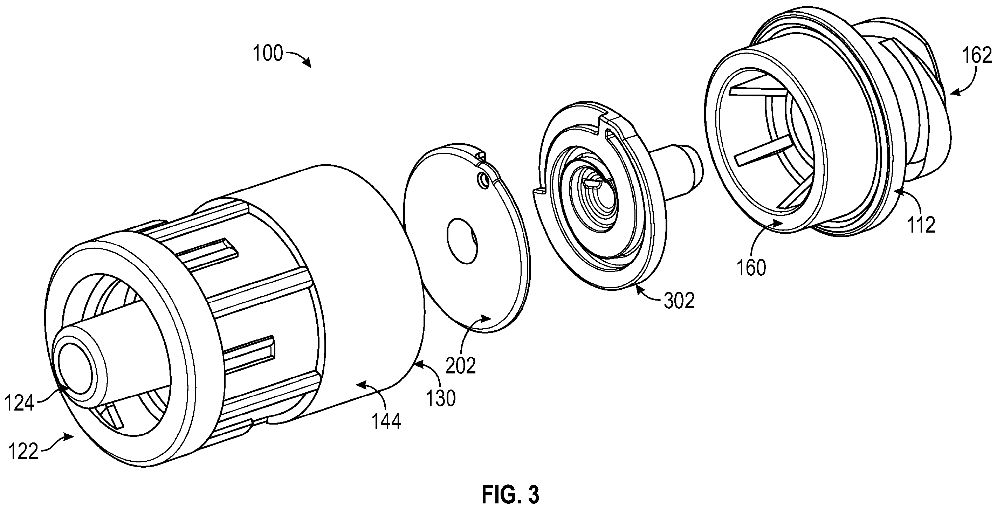

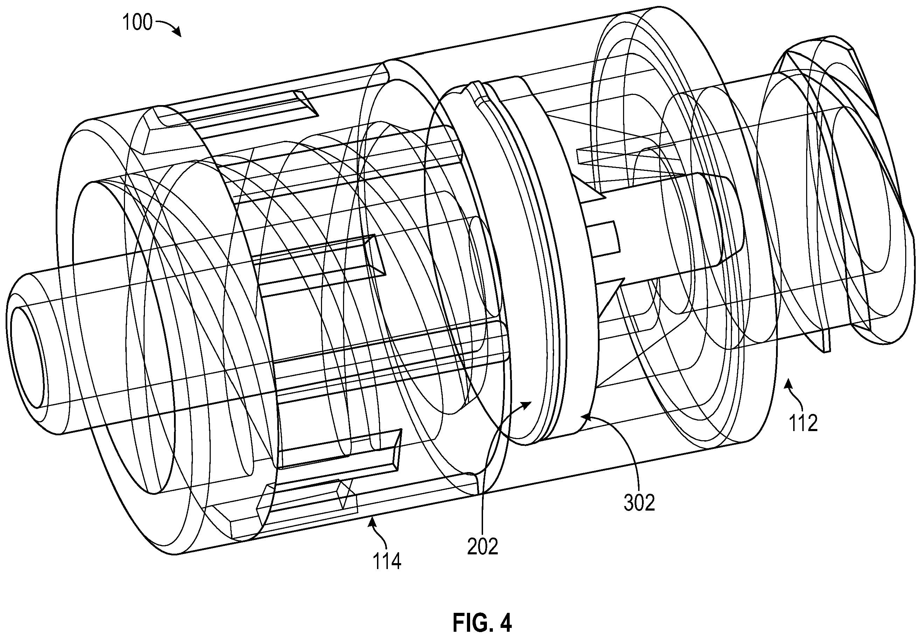

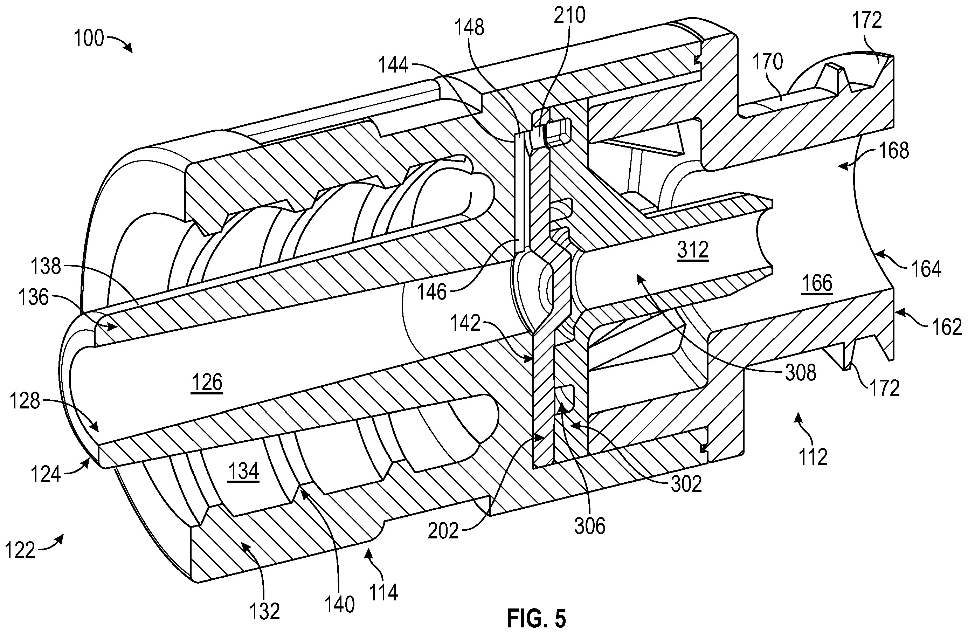

FIG. 2 illustrates a perspective view of a flow restriction device 100 having a proximal housing 112 and a distal housing 114, in accordance with some embodiments of the present disclosure. FIG. 3 illustrates an exploded view of the flow restriction device 100 having a proximal housing 112, a distal housing 114, a resilient valve 202, and a flow insert 302, and where the resilient valve 202 and the flow insert 302, are configured to be positioned between the proximal and distal housings 112, 114. FIG. 4 illustrates a perspective view of the flow restriction device 100 with the proximal and distal housings 112, 114 shown as being transparent to illustrate the resilient valve 202 and the flow insert 302 positioned within a cavity between the proximal and distal housings 112, 114. FIG. 5 illustrate a cross-sectional view of the flow restriction device 100 of FIG. 2, along line 5-5.

As illustrated in FIGS. 2-5, in some embodiments, the flow restriction device 100 includes a proximal housing 112 and a distal housing 114. Each of the proximal and distal housings 112, 114 include a port configured to couple a fluid flow path of the flow restriction device 100 with other devices, such as a vascular access device 10, a blood collection device 40, and/or a catheter assembly 50. In some embodiments of the present disclosure, the distal housing 114 can include a first end portion 122 forming a distal port 124 of the flow restriction device, and the proximal housing 112 can include a second end portion 162 forming a proximal port 164 of the flow restriction device.

The distal port 124 can be configured to couple with a blood collection device, such a vascular access device 10 or a catheter assembly 50, and the proximal port 164 can be configured to couple with a blood collection device 40 such as a syringe. In some embodiments of the present disclosure, any of the proximal and distal ports 164, 124 can form any of a male or female luer connector. In some embodiments, the proximal port 164 is formed as female luer connector and the distal port 124 is formed as a male luer connector.

The distal port 124 can be formed by an inner surface 126 of the distal housing 114. The inner surface 126 defines a passage 128 through the distal housing, with the passage extending from the first end portion 122 to a second end portion 130 of the distal housing.

The distal housing 114 can also include, in some embodiments, a wall 132 forming the first end portion 122 and having an inner surface 134 that forms a female connector. The distal housing 114 can also include a protrusion 136 that extends along the first end portion 122 of the distal housing and is positioned radially inward from the inner surface 134 of the wall such that an outer surface 138 of the protrusion is spaced apart from the inner surface 134 of the wall.

The protrusion 136 and the wall 132 form a connector of the flow restriction device configured to couple with another device positioned between the inner surface 134 of the wall and the outer surface 138 of the protrusion, where the passage 128 of the distal housing extends through the protrusion 136.

In some embodiments of the present disclosure, the inner surface 134 of the wall can include a fastening structure configured to engage against and/or couple the flow restriction device 100 with another device. In some aspects, the fastening structure of the distal housing 114 can be a thread 140 that extends along the inner surface 134 of the wall. In some examples of the present disclosure, the fastening structure of the distal housing 114 can be the inner surface 134 of the wall configured to engage against another device to form an interference fit therebetween.

The second end portion 130 of the distal housing has an inner surface forming a bore that extends into the distal housing, in a direction from the second end portion 130 toward the first end portion 122, to a recessed surface 142. The passage 128 of the proximal housing extends through the recessed surface 142 into the bore.

The second end portion 130 of the distal housing can also include a flow channel 144 configured to be fluidly coupled with the passage 128. The flow channel 144 extends into the recessed surface 142 to define another portion of the fluid flow path through the flow restriction device 100.

The flow channel 144 includes a first or proximal end 146 that that intersects the passage 128 of the distal housing to fluidly couple the flow channel 144 with the passage 128. The flow channel 144 extends in a direction away or radially outward from the passage 128. The flow channel 144 also includes a second or distal end 148 that is configured to fluidly couple with another portion of the fluid flow path of the flow restriction device 100.

The proximal housing 112 is configured to couple with the distal housing 114 to form another portion of the fluid flow path of the flow restriction device 100. The fluid flow path of the proximal housing 112 can include the proximal port 164. The proximal port is formed by an inner surface 166 of the proximal housing 112. The inner surface 166 defines a passage 168 through the proximal housing, with the passage extending from a first end portion 160 to the second end portion 162 of the proximal housing.

In some embodiments of the present disclosure the first end portion 160 of the proximal housing has an inner surface forming a bore that extends into the proximal housing, in a direction from the first end portion 160 toward the second end portion 162.

The proximal housing 112 and the distal housing 114 are configured to form a cavity therebetween, where the cavity is configured to receive a resilient valve 202 and a flow insert 302. In some embodiments of the present disclosure, the resilient valve 202 and the flow insert 302 are positioned between the recessed surface 142 of the distal housing and the first end portion 160 of the proximal housing. In some embodiments, the resilient valve 202 and the flow insert 302 are positioned in the cavity formed between the proximal and distal housings 112, 114.

In some embodiments of the present disclosure, an outer surface 170 of the second end portion of the proximal housing can include a fastening structure configured to engage against and/or couple the flow restriction device 100 with another device. In some aspects, the fastening structure of the proximal housing 112 can be a thread 172 that extends along the outer surface 170 of the second end portion.

The resilient valve 202 is positioned between the proximal and distal housings 112, 114, and is configured to resist movement of a fluid along the fluid flow path of the flow restriction device 100. The resilient valve 202, which is illustrated in FIGS. 3-7, includes a first end 203 and a second end 204 where the second end 204 is opposite to the first end 203. Additionally, an inner portion 206 and an outer portion 208 that extends around the inner portion 206 is defined along the first and second ends 203, 204 of the resilient valve. The inner portion 206 is flexible, relative to the outer portion 208, so that the inner portion 206 can move or bias when a force or pressure is exerted on the resilient valve.

In some embodiments of the present disclosure, the first end 203 of the resilient valve forms a convex surface along the inner portion 206, and the second end 204 of the resilient valve forms a concave surface along the inner portion 206. When the resilient valve 202 is positioned between the proximal and distal housings 112, 114, the inner portion 206 along the second end 204 is aligned with the passage 128 of the distal housing, and the inner portion along the first end 203 is aligned with another fluid passage of the flow restriction device 100.

The resilient valve 202, in some embodiments, is shaped as a disk having a radial center 211 and an outer perimeter 212 that extends around the center. In some aspects, the inner portion 206 is aligned with the center 211 of the resilient valve, and the outer portion 208 extends from the inner portion to the outer perimeter 212 of the resilient valve.

The resilient valve 202 includes an aperture 210 that extends through the first and second ends 203, 204, and is configured to form a portion of the fluid flow path of the flow restriction device 100. The aperture 210 forms a portion of the fluid flow path that permits a fluid to move through the resilient valve 202.

The aperture 210 can be located along the outer portion 208, however, it should be understood that the present disclosure contemplates the aperture 210 positioned along any of the inner and/or outer portions 206, 208. In some embodiments of the present disclosure, a portion of the fluid flow path extends along any of the outer surface of the first end 203, the second end 204, the outer perimeter 212, and/or the aperture 210.

To align the resilient valve 202 with another portion of the flow restriction device 100, the resilient valve 202 can include an alignment ridge 214. In some embodiments, the alignment ridge 214 extends from the surface of the first end 203, in a direction away from the second end 204. The alignment ridge 214 can be configured to that when the resilient valve 202 is positioned next to the flow insert 302, the alignment ridge 214 can engage against or be positioned within a complementary feature of the flow insert 302. In some aspects, when the resilient valve 202 is positioned next to the flow insert 302, and the alignment ridge 214 is positioned within or engaged against a complementary feature of the flow insert 302, the aperture 210 of the resilient valve is aligned with and/or intersects a portion of the fluid flow path formed by the flow insert 302.

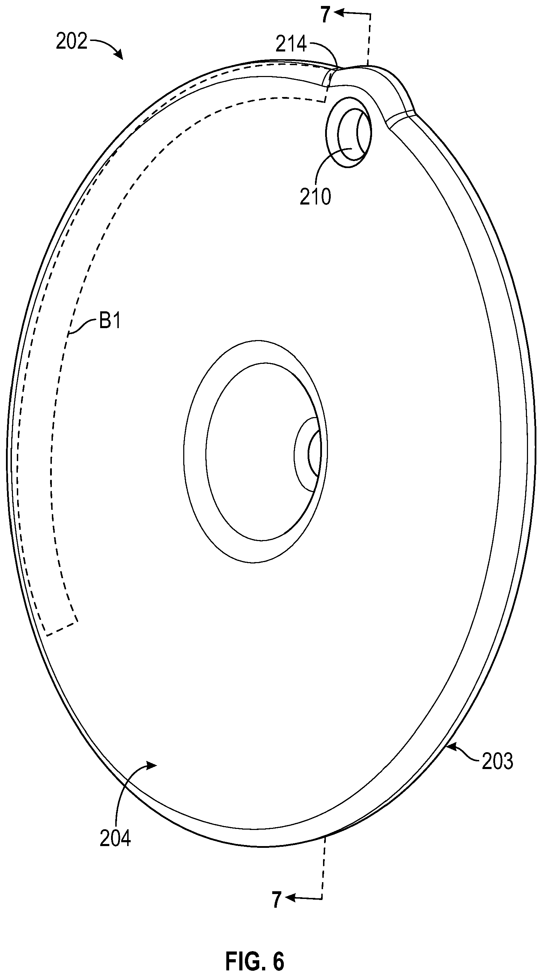

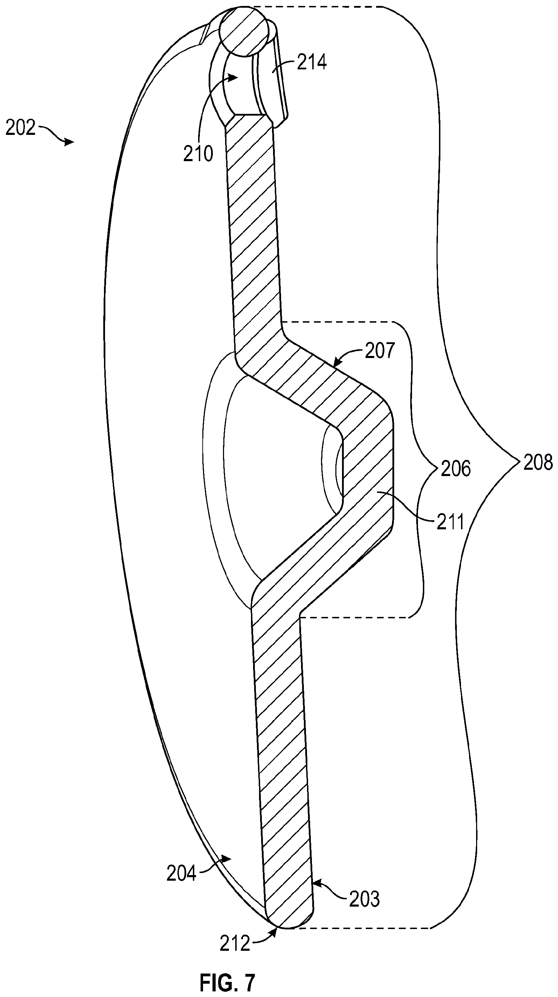

Referring to FIG. 7, the alignment ridge 214 is illustrated extending from the first end 203 of the resilient valve, in a direction away from the second end 204 of the resilient valve. The alignment ridge 214 is shaped as an elongate wall having a width and a length. The width of the alignment ridge 214 extends in a direction from the center 211 toward the outer perimeter 212 of the resilient valve, and the length of the alignment ridge 214 extends in a direction along the outer perimeter 212 of the resilient valve. The shape of the alignment ridge 214 along the first end 203 of the resilient valve is illustrated with a broken line B1 along the second end 204 of the resilient valve in FIG. 6 for reference.

It should be understood that although the alignment ridge 214 is illustrated as extending away from the first end 203 of the resilient valve, the present application contemplates that an alignment ridge can also be shaped as any of a concave surface and/or convex surface that extends from any of the first and/or second ends 203, 204 of the resilient valve. In some embodiments of the present disclosure the alignment ridge can be any of a protrusion and/or indentation along the outer perimeter 212 of the resilient valve.

The resilient valve 202 is positioned adjacent to the flow insert 302 to form another portion of the fluid flow path between the resilient valve 202 and the flow restriction device 100. To form the portion of the fluid flow path of the flow restriction device 100, the flow insert 302 has an inner surface forming a fluid passage therethrough. The fluid passage includes a first segment 306 and a second segment 308. The first segment 306 is formed by an outer surface 310 of the flow insert, and the second segment 308 is formed by an inner surface 312 of the flow insert.

In some embodiments of the present disclosure, the outer surface 310 is formed by a distal-most end surface of the flow insert 302 that is configured to engage against at least a portion of the resilient valve 202. The outer surface 310 can be shaped as a planar surface configured to engage against the resilient valve to form a portion of the flow path therebetween.

The first segment 306 is formed by a channel that extends into the outer surface 310, and along a direction that extends from an outer circumferential portion of the flow insert toward a longitudinal axis BB defined by the second segment 308 of the passage. The outer surface 310 can define a plane that is transverse, relative to the longitudinal axis BB.

The first segment 306 of the fluid passage for the flow insert extends along the outer surface 310 in a direction that is non-linear, such that the first segment 306 forms a spiral or involute shape in the direction toward the longitudinal axis BB. The first segment 306 of the passage can also be defined by a first end 313 located adjacent to the outer circumferential portion of the flow insert, and a second end 314 that intersects the second segment 308 of the passage.

The first segment 306 of the fluid passage can have a length between the first and second ends 313, 314, and a width that is transverse to the length. In some embodiments of the present disclosure, the length of the first segment 306 is approximately 1 inch (25.4 mm), and the width of the first segment 306 is approximately 0.02 inch (0.51 mm).

In some embodiment of the present disclosure, a portion of the fluid passage of the flow insert 302 is formed by a groove 316 that extends into the outer surface 310 of the flow insert, and extends between the first and second segments 306, 308 of the fluid passage. The groove 316 can be configured to permit a fluid to move between the first and second segments 306, 308 of the fluid passage when the resilient valve 202 moves or is biased toward the flow insert 302.

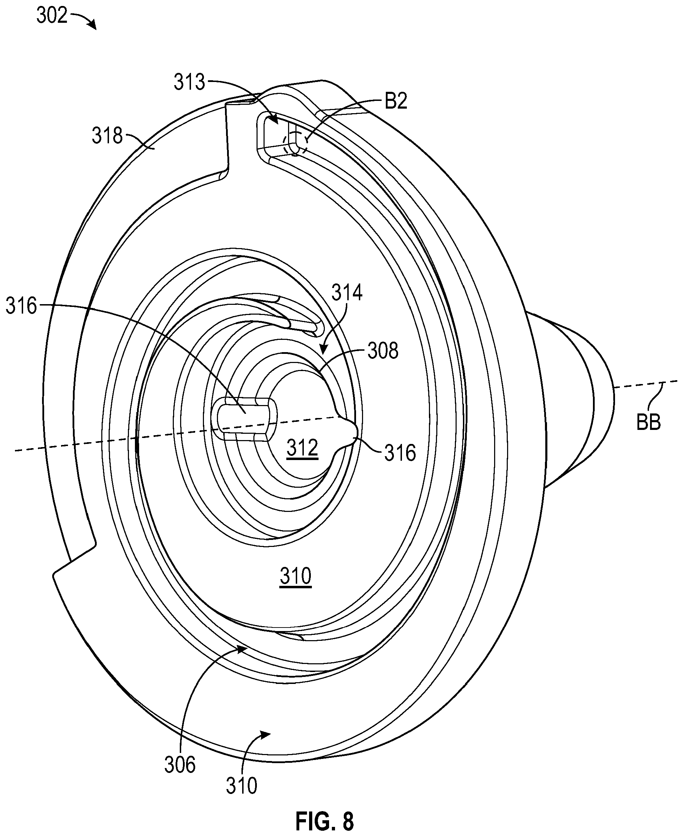

The flow insert 302 can also include an alignment channel 318 configured to receive the alignment ridge 214 of the resilient valve therein. In some aspects of the present disclosure, the alignment ridge 214 and the alignment channel 318 are positioned on the resilient valve 202 and the flow insert 302, respectively, so that the aperture 210 of the resilient valve is aligned with the first segment 306 of the passage, as illustrated by the broken line B2 in FIG. 8.

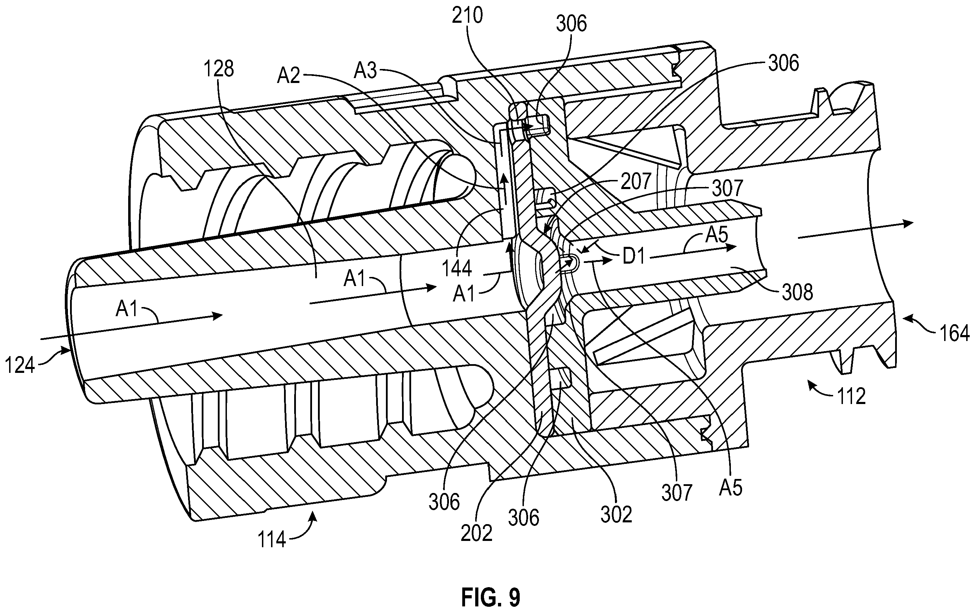

Referring to FIG. 9, and with continued reference to FIGS. 5-8, a cross-sectional view of the flow restriction device 100 is illustrated with the resilient valve 202 in a first position between the proximal and distal housings 112, 114. The resilient valve 202 is oriented with the second end 204 adjacent to or engaging against the recessed surface 142 of the distal housing, and the first end 203 adjacent to or engaging against the outer surface 310 of the flow insert.

Along the second end 204 of the resilient valve, the inner portion 206 is aligned with the passage 128 of the distal housing 114, and the outer portion 208 is aligned with the flow channel 144. The space formed along the flow channel 144 of the distal housing and the resilient valve 202 defines a portion of the fluid flow path. It should be understood that in some embodiments of the present disclosure, the flow channel can extend into the resilient valve to form a space between the resilient valve and the distal housing defining a portion of the fluid flow path.

The aperture 210 of the resilient valve is aligned with the flow channel 144 of the distal housing to permit a fluid to move through the resilient valve in a direction toward or away from the flow channel 144. The aperture 210 of the resilient valve can aligned with the distal end 148 of the flow channel or along another portion of the flow channel 144. In some embodiments of the present disclosure, the aperture 210 is aligned with the distal end 148 of the flow channel when the alignment ridge 214 of the resilient valve is engaged against or positioned within the alignment channel 318 of the flow insert.

Along the first end 203 of the resilient valve, the outer portion 208 and the aperture 210 are aligned with the first segment 306 of the passage, so that a fluid can move between the flow channel 144 and the first segment 306 of the passage by moving through the aperture 210.

It should be understood that in some embodiments of the present disclosure, the aperture 210 can be formed as a channel or groove along the outer perimeter 212 of the resilient valve. Also, in some embodiments of the present disclosure, a portion of the fluid flow path extends around the outer perimeter 212 of the resilient valve instead of or in addition to extending through the resilient valve.

The inner portion 206 of the resilient valve is aligned with the second segment 308 of the passage to form a portion of the fluid flow path therebetween. When the resilient valve 202 is in the first position, the inner portion 206 of the resilient valve is spaced apart from the flow insert by a distance D1 to permit a fluid to move along the fluid flow path between the resilient valve 202 and the flow insert 302. In some embodiments of the present disclosure the convex surface of the inner portion 206 defines a shoulder 207 configured to engage against a valve seat 307 of the flow insert, where the valve seat 307 is between the first and second segments 306, 308 of the passage.

The fluid flow path through the flow restriction device 100, when the resilient valve 202 is in the first position, is illustrated in FIG. 9. A fluid, such as blood, can move through the distal port 124 of distal housing (Arrow A1). The fluid moves along the passage 128 toward the flow channel 144 of the distal housing. The fluid can then move along the flow channel 144 (Arrow A2) toward the aperture 210 of the resilient valve 202. The fluid moves through the aperture 210 (Arrow A3) and enters into the first segment 306 of the fluid passage for the flow insert 302.

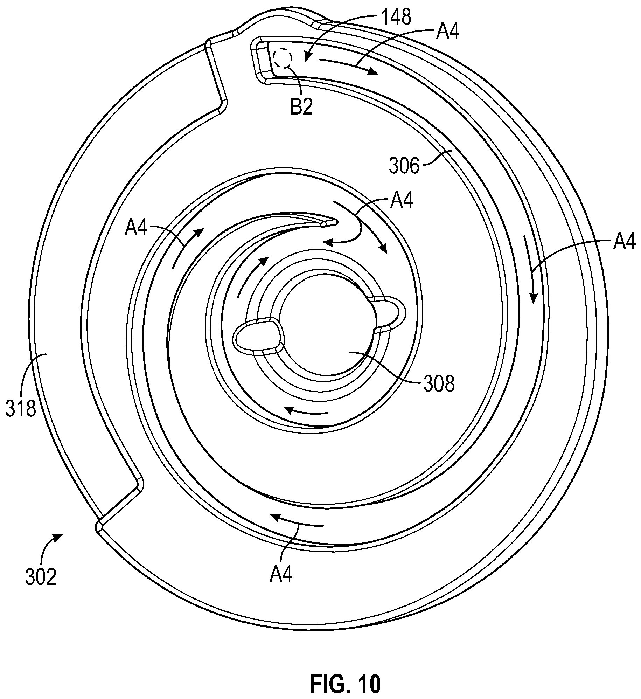

Referring to FIGS. 9 and 10, the fluid can enter the first end 313 of the first segment 306 of the fluid passage, the location illustrated by way of reference with broken line B2. The fluid then moves along the first segment 306 along a spiral or involute shaped path toward the second segment 308 of the passage.

When the resilient valve 202 is in the first position, the fluid can move between the inner portion 206 of the resilient valve and the flow insert 302, and into the second segment 308 of the passage. Thereafter, the fluid moves along the second segment 308 (Arrow A5) toward the proximal port 164.

The resilient valve 202 is configured to respond to a change in pressure in the fluid flow path through the flow restriction device 100. For example, the resilient valve 202, or a portion thereof, can respond to a change in pressure in the fluid flow path by moving or biasing relative to other portions of the resilient valve or flow restriction device 100. The change in pressure in the fluid flow path can exert a pressure on the resilient valve 202, which causes the resilient valve 202 to bias or stretch.

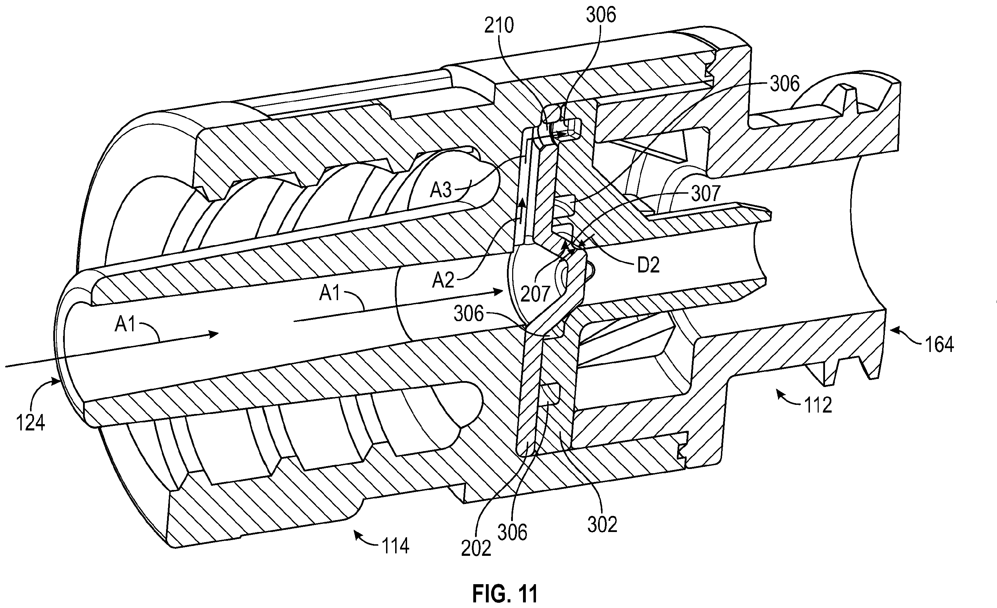

When a suction pressure at the proximal port 164 exceeds a certain value or range, the inner portion 206 of the resilient valve is biased or stretches toward the flow insert 302 such that the resilient valve 202 is in the second position. The second position of the resilient valve 202 is illustrated in FIG. 11.

In the second position, the resilient valve 202 has biased or stretched toward the flow insert 302 to resist or stop the flow rate of the fluid through the fluid flow path of the flow restriction device 100. In some embodiments of the present disclosure, the shoulder 207 of the resilient valve is spaced apart from the valve seat 307 of the flow insert by a distance D2, where distance D2 is less than D1 when the resilient valve 202 is in the second position. In some embodiments of the present disclosure, the distance D2 is zero such that movement of a fluid through the flow restriction device 100 is resisted.

When the resilient valve 202 is in the second position, the flow rate of the fluid through the fluid flow path is reduced or stopped and the pressure on the fluid is thereby reduced. When the fluid moving through the fluid flow path is blood, the reduction of pressure can reduce homolysis to the blood.

In some embodiments of the present disclosure, the resilient valve 202 moves from the first position toward the second position when a pressure along the fluid flow path exerts a force of approximately 0.03 Newtons on the resilient valve 202. In some embodiments, the resilient valve 202 is configured so that portion of the resilient valve, such as the inner portion 206, is displaced or biased between approximately 0.03 mm to approximately 0.1 mm when a force of approximately 0.03 Newtons is exerted on the resilient valve 202. In some embodiments, the resilient valve 202 is configured so that portion of the resilient valve, such as the inner portion 206, is displaced or biased between approximately 0.1 mm to approximately 0.15 mm when a force of approximately 0.09 Newtons is exerted on the resilient valve 202.

In some aspects of the present disclosure, the resilient valve 202, or a portion thereof, is comprised of an elastomer material, such as silicone. Any of the proximal and distal housings 112, 114, and the flow insert 302 can comprise a thermoplastic polymer material, such as polycarbonate.

## Claims

1. A flow restriction device (100), comprising:
a flow insert (302) comprising a fluid passage extending therethrough, the fluid passage comprising a first segment (306) formed by an outer surface (310) of the flow insert, and a second segment (308) formed by an inner surface (312) of the flow insert;
a resilient valve (202) comprising a first end (203) engaged against the outer surface (310) of the flow insert, the first end (203) having an inner portion (206) aligned with the second segment (308) of the fluid passage, and an outer portion (208) aligned with the first segment (306) of the fluid passage, and an aperture (210) that extends through the resilient valve, from the first end (203) to a second end of the resilient valve;
wherein the inner portion (206) of the resilient valve is flexible, relative to the flow insert (302) such that in a first orientation of the resilient valve (202), the inner portion (206) is spaced apart from the flow insert by a first distance (D1) to permit a fluid to move from the first segment (306) toward the second segment (308) of the fluid passage, and in a second orientation of the resilient valve (202), the inner portion (206) is spaced apart from the flow insert (302) by a second distance (D2) to resist movement of the fluid between from the first segment (306) to the second segment (308) of the fluid passage.

2. The flow restriction device of Claim 1, wherein the second segment (308) of the fluid passage defines an axis (BB) therethrough, and the first segment (306) of the fluid passage extends around the axis.

3. The flow restriction device of Claim 1, wherein the first segment (306) of the fluid passage extends radially outward in a spiral direction away from the second segment (308) of the fluid passage.

4. The flow restriction device of Claim 1, wherein the first segment (306) of the fluid passage is formed by a channel that extends into the outer surface (310) of the flow insert.

5. The flow restriction device of Claim 4, wherein the outer surface (310) of the flow insert extends along a plane that is transverse relative to an axis (BB) through the second segment (308) of the fluid passage.

6. The flow restriction device of Claim 1, wherein the flow insert comprises a groove (316) that extends into the outer surface (310) of the flow insert, between the first and second segments (306, 308) of the fluid passage.

7. The flow restriction device of Claim 1, wherein the first end (203) of the resilient valve forms a convex surface along the inner portion (206), and the second end of the resilient valve forms a concave surface along the inner portion (206).

8. The flow restriction device of Claim 1, wherein the aperture (210) extends through the outer portion (208) of the resilient valve.

9. The flow restriction device of Claim 1, wherein the aperture (210) of the resilient valve is aligned with the first segment (306) of the fluid passage.

10. The flow restriction device of Claim 1, wherein the resilient valve comprises an alignment ridge (214) that extends from the first end (203) in a direction away from the second end.

## Patentansprüche

1. Eine Durchflussbegrenzungsvorrichtung (100), umfassend:
einen Durchflusseinsatz (302) mit einem sich durch diesen erstreckenden Fluidkanal, wobei der Fluidkanal ein erstes Segment (306), das durch eine Außenfläche (310) des Durchflusseinsatzes gebildet ist, und ein zweites Segment (308) umfasst, das durch eine Innenfläche (312) des Durchflusseinsatzes gebildet ist;
ein elastisches Ventil (202) mit einem ersten Ende (203), das an der Außenfläche (310) des Durchflusseinsatzes anliegt, wobei das erste Ende (203) einen inneren Abschnitt (206), der mit dem zweiten Segment (308) des Fluidkanals fluchtet, und einen äußeren Abschnitt (208), der mit dem ersten Segment (306) des Fluidkanals fluchtet, und eine Öffnung (210) aufweist, die sich durch das elastische Ventil von dem ersten Ende (203) zu einem zweiten Ende des elastischen Ventils erstreckt;
wobei der innere Abschnitt (206) des elastischen Ventils relativ zum Durchflusseinsatz (302) flexibel ist, so dass in einer ersten Ausrichtung des elastischen Ventils (202) der innere Abschnitt (206) um einen ersten Abstand (D1) vom Durchflusseinsatz beabstandet ist, um einem Fluid zu ermöglichen, sich vom ersten Segment (306) zum zweiten Segment (308) des Fluidkanals strömen kann, und in einer zweiten Ausrichtung des elastischen Ventils (202) der innere Abschnitt (206) um einen zweiten Abstand (D2) vom Durchflusseinsatz (302) beabstandet ist, um einer Bewegung des Fluids zwischen dem ersten Segment (306) und dem zweiten Segment (308) des Fluidkanals entgegenzuwirken.

2. Die Durchflussbegrenzungsvorrichtung nach Anspruch 1, wobei das zweite Segment (308) des Fluidkanals eine Achse (BB) durch sich hindurch definiert und das erste Segment (306) des Fluidkanals sich um die Achse herum erstreckt.

3. Die Durchflussbegrenzungsvorrichtung nach Anspruch 1, wobei sich das erste Segment (306) des Fluidkanals radial nach außen in einer spiralförmigen Richtung vom zweiten Segment (308) des Fluidkanals weg erstreckt.

4. Die Durchflussbegrenzungsvorrichtung nach Anspruch 1, wobei das erste Segment (306) des Fluidkanals durch einen Kanal gebildet ist, der sich in die Außenfläche (310) des Durchflusseinsatzes erstreckt.

5. Die Durchflussbegrenzungsvorrichtung nach Anspruch 4, wobei sich die Außenfläche (310) des Durchflusseinsatzes entlang einer Ebene erstreckt, die quer zu einer Achse (BB) durch das zweite Segment (308) des Fluidkanals verläuft.

6. Die Durchflussbegrenzungsvorrichtung nach Anspruch 1, wobei der Durchflusseinsatz eine Nut (316) umfasst, die sich in die Außenfläche (310) des Durchflusseinsatzes zwischen dem ersten und dem zweiten Segment (306, 308) des Fluidkanals erstreckt.

7. Die Durchflussbegrenzungsvorrichtung nach Anspruch 1, wobei das erste Ende (203) des elastischen Ventils eine konvexe Fläche entlang des inneren Abschnitts (206) bildet und das zweite Ende des elastischen Ventils eine konkave Fläche entlang des inneren Abschnitts (206) bildet.

8. Die Durchflussbegrenzungsvorrichtung nach Anspruch 1, wobei sich die Öffnung (210) durch den äußeren Abschnitt (208) des elastischen Ventils erstreckt.

9. Die Durchflussbegrenzungsvorrichtung nach Anspruch 1, wobei die Öffnung (210) des elastischen Ventils mit dem ersten Segment (306) des Fluidkanals ausgerichtet ist.

10. Die Durchflussbegrenzungsvorrichtung nach Anspruch 1, wobei das elastische Ventil einen Ausrichtungssteg (214) umfasst, der sich vom ersten Ende (203) in einer Richtung weg vom zweiten Ende erstreckt.

## Revendications

1. Dispositif de restriction du débit (100), comprenant :
un insert d'écoulement (302) comprenant un passage de fluide s'étendant à travers lui, le passage de fluide comprenant un premier segment (306) formé par une surface extérieure (310) de l'insert d'écoulement, et un deuxième segment (308) formé par une surface intérieure (312) de l'insert d'écoulement ;
une valve résiliente (202) comprenant une première extrémité (203) engagée contre la surface extérieure (310) de l'insert d'écoulement, la première extrémité (203) ayant une partie interne (206) alignée avec le deuxième segment (308) du passage de fluide, et une partie extérieure (208) alignée avec le premier segment (306) du passage de fluide, et une ouverture (210) qui s'étend à travers la valve résiliente, de la première extrémité (203) à une deuxième extrémité de la valve résiliente ;
dans laquelle la partie interne (206) de la valve résiliente est flexible par rapport à l'insert d'écoulement (302) de sorte que dans une première orientation de la valve résiliente (202), la partie interne (206) est espacée de l'insert d'écoulement d'une première distance (D1) pour permettre à un fluide de se déplacer du premier segment (306) vers le deuxième segment (308) du passage de fluide, et dans une deuxième orientation de la valve résiliente (202), la partie interne (206) est espacée de l'insert d'écoulement (302) d'une deuxième distance (D2) pour résister au mouvement du fluide entre le premier segment (306) et le deuxième segment (308) du passage de fluide.

2. Le dispositif de restriction du débit de la revendication 1, dans lequel le deuxième segment (308) du passage de fluide définit un axe (BB) à travers lui, et le premier segment (306) du passage de fluide s'étend autour de l'axe.

3. Le dispositif de restriction du débit de la revendication 1, dans lequel le premier segment (306) du passage de fluide s'étend radialement vers l'extérieur dans une direction en spirale loin du deuxième segment (308) du passage de fluide.

4. Le dispositif de restriction du débit de la revendication 1, dans lequel le premier segment (306) du passage de fluide est formé par un canal qui s'étend dans la surface extérieure (310) de l'insert d'écoulement.

5. Le dispositif de restriction du débit de la revendication 4, dans lequel la surface extérieure (310) de l'insert d'écoulement s'étend le long d'un plan qui est transversal par rapport à un axe (BB) à travers le deuxième segment (308) du passage de fluide.

6. Le dispositif de restriction du débit de la revendication 1, dans lequel l'insert de débit comprend une rainure (316) qui s'étend dans la surface extérieure (310) de l'insert de débit, entre le premier et le deuxième segment (306, 308) du passage de fluide.

7. Le dispositif de restriction du débit de la revendication 1, dans lequel la première extrémité (203) de la valve résiliente forme une surface convexe le long de la partie interne (206), et la deuxième extrémité de la valve résiliente forme une surface concave le long de la partie interne (206).

8. Le dispositif de restriction du débit de la revendication 1, dans lequel l'ouverture (210) s'étend à travers la partie extérieure (208) de la valve résiliente.

9. Le dispositif de restriction du débit de la revendication 1, dans lequel l'ouverture (210) de la valve résiliente est alignée avec le premier segment (306) du passage de fluide.

10. Le dispositif de restriction du débit de la revendication 1, dans lequel la valve résiliente comprend une crête d'alignement (214) qui s'étend de la première extrémité (203) dans une direction opposée à la deuxième extrémité.
